# EUROPÄISCHE PATENTANMELDUNG

(11) **EP 0 560 399 A1**
(43) Veröffentlichungstag der Anmeldung: **15.09.1993**
(21) Anmeldenummer: 93105146.0
(22) Anmeldetag: 17.07.1989
(51) Int. Cl.: A61K 31/40, A61K 31/445, A61K 31/425, A61K 31/495, A61K 31/535

(54) **Verwendung substituierter 3,4-Dihydro-2H-benzopyrane als Heilmittel gegen Störungen der ableitenden Harnwege**

(30) Priorität: 19.07.1988 DE 3824446
(62) Teilanmeldung aus: 89112897.7
(71) Anmelder: HOECHST AKTIENGESELLSCHAFT, D-65926 Frankfurt am Main (DE)
(72) Erfinder: Englert, Heinrich, Dr., W-6238 Hofheim/Ts. (DE); Klaus, Erik, Dr., W-6233 Kelkheim/Ts. (DE); Mania, Dieter, Dr., W-6240 Königstein/Ts. (DE); Schölkens, Bernward, Prof. Dr., W-6233 Kelkheim/Ts. (DE)

(57) **Zusammenfassung**

Beschrieben wird die Verwendung von 3,4-Dihydro-2H-benzo[b]pyranen der Formel I 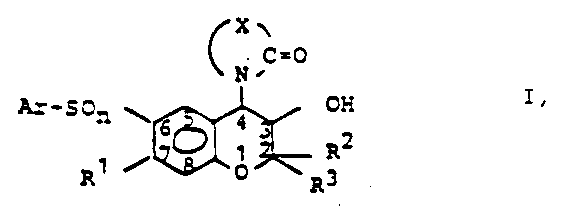
in welcher
- R¹: für H, OH, (C₁-C₂)-Alkoxy, (C₁-C₂)-Alkyl oder NR⁴R⁵ steht, wobei R⁴ und R⁵ gleich oder verschieden sind und für H, (C₁-C₂)Alkyl oder (C₁-C₃)-Alkylcarbonyl stehen,
- R²,R³: gleich oder verschieden sind und für Alkyl mit 1-4 C-Atomen stehen,
- Ar: für ein aromatisches oder heteroaromatisches System steht, das unsubstituiert oder durch 1 bis 3 gleiche oder verschiedene Reste (C₁-C₂)-Alkyl, (C₁-C₂)Alkoxy, Halogen, Trifluormethyl, CN, NO₂, CO-(C₁-C₂)Alkyl oder SOₘ-(C₁-C₂)-Alkyl mit m= 1 oder 2 substituiert ist,
- n: für 1 oder 2 steht,
- X: für eine Kette (CH₂)ᵣ steht, die durch ein Heteroatom 0, S oder NR⁶ unterbrochen sein kann, wobei R⁶ H oder (C₁-C₄)-Alkyl bedeutet und r für die Zahlen 2, 3, 4 oder 5 steht,
zur Herstellung eines Heilmittels gegen Störungen der ableitenden Harnwege.

## Beschreibung

Die Erfindung betrifft die Verwendung von 3,4-Dihydro-2H-benzo[b]pyranen der Formel I 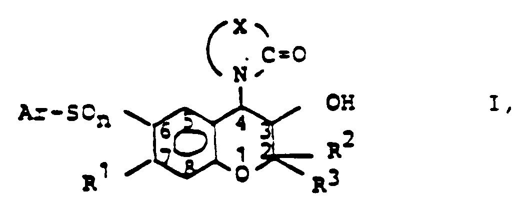
in welcher
- R¹: für H, OH, (C₁-C₂)-Alkoxy, (C₁-C₂)-Alkyl oder NR⁴R⁵ steht, wobei R⁴ und R⁵ gleich oder verschieden sind und für H, (C₁-C₂)Alkyl oder (C₁-C₃)-Alkylcarbonyl stehen,
- R²,R³: gleich oder verschieden sind und für Alkyl mit 1-4 C-Atomen stehen,
- Ar: für ein aromatisches oder heteroaromatisches System steht, das unsubstituiert oder durch 1 bis 3 gleiche oder verschiedene Reste (C₁-C₂)-Alkyl, (C₁-C₂)Alkoxy, Halogen, Trifluormethyl, CN, NO₂, CO-(C₁-C₂)Alkyl oder SOₘ-(C₁-C₂)-Alkyl mit m= 1 oder 2 substituiert ist,
- n: für 1 oder 2 steht,
- X: für eine Kette (CH₂)ᵣ steht, die durch ein Heteroatom 0, S oder NR⁶ unterbrochen sein kann, wobei R⁶ H oder (C₁-C₄)-Alkyl bedeutet und r für die Zahlen 2, 3, 4 oder 5 steht,
zur Herstellung eines Heilmittels gegen Störungen der ableitenden Harnwege.

Unter einem aromatischen System Ar wird vorzugsweise Phenyl, Naphthyl oder Biphenylyl verstanden, ein 5- oder 6-gliedriges heteroaromatisches System Ar ist vorzugsweise ein Rest eines 5- oder 6-gliedrigen O, N und/oder S-heterocyclischen Ringes, insbesondere Furyl, Thienyl, Isothiazolyl, Oxazolyl, Isoxazolyl, Pyrazolyl, Imidazolyl, Thiazolyl, Pyridyl, Pyrazinyl, Pyrimidinyl, Pyridazinyl, Triazinyl.

Unter Halogen wird F, Cl, Br oder J, vorzugsweise F und Cl verstanden.

Die C-Atome 3 und 4 des 3,4-Dihydro-2H-benzo[b]pyransystems (nachfolgend auch kurz "Chromansystem" genannt) der Formel I sind asymmetrisch substituiert. Gegenstand der Erfindung sind dabei nur solche Verbindungen, die an diesen Zentren entgegengesetzte Konfigurationen, also eine "trans"-Orientierung der Substituenten an diesen C-Atomen aufweisen. Enthält einer der Substituenten R¹, ArSOₙ, R² und/oder R³ Asymmetriezentren oder, falls R² und R³ ungleich sind (und somit ein asymmetrisches Kohlenstoffatom erzeugen), sind Verbindungen mit sowohl S- als auch R-konfigurierten Zentren Gegenstand der Erfindung.

Die Verbindungen können als optische Isomere, als Diastereoisomere, als Racemate oder als Gemische derselben vorliegen.

Bevorzugt werden Verbindungen der Formel I verwendet, bei denen R¹ bis R³, ArSOₙ die oben erwähnten Bedeutungen haben, X jedoch für eine Kette (CH₂)ᵣ steht mit r= 3 oder 4.

Ganz besonders bevorzugt verwendet werden solche Verbindungen, bei denen R¹ bis R³ die oben erwähnten Bedeutungen haben, Ar für Phenyl steht, das unsubstituiert oder wie oben definiert substituiert ist, n für 2 steht und X für eine Kette (CH₂)ᵣ mit r= 3 oder 4.

Insbesondere bevorzugte Verwendung finden solche Verbindungen, bei denen R¹ H bedeutet, R² und R³ für (C₁-C₂)Alkyl stehen, Ar für Phenyl steht, das unsubstituiert oder durch (C₁-C₂)-Alkyl, (C₁-C₂)-Alkoxy oder Halogen einfach substituiert ist, n für 2 steht und X für eine Kette (CH₂)ᵣ mit r= 3 oder 4.

Ebenfalls bevorzugt sind Verbindungen mit R¹ gleich H, R² und R³ gleich (C₁-C₂)-Alkyl, X gleich (CH₂)₃, Ar gleich C₆H₄Cl und n gleich 2, sowie solche mit R¹ gleich H,
R² und R³ gleich (C₁-C₂)-Alkyl, Ar gleich Phenyl, n gleich 2 und X gleich (CH₂)₃.

EP 0 176 689 beschreibt die Verwendung von Benzopyranen bei Atemwegskrankheiten und/oder Störungen des Gastrointestinaltrakts und/oder des Uterus, wobei insbesondere solche Störungen hervorgehoben werden, die bei glattmuskulären Kontraktionen auftreten.
EP 207 614 beschreibt die Verwendung von Benzopyranen bei der Inkontinenz. EP 277 611 beschreibt die Verwendung von Benzopyranen, die stets einen Methyl-Substituenten im Pyrrolidin-Ring enthalten, zur Herstellung eines Arzneimittels gegen Störungen der ableitenden Harnwege.
Ferner ist aus J. Med. Chem. 1986, 29, 2194 - 2201 bekannt daß derartige Verbindungen blutdrucksenkende Eigenschaften haben können.
Überraschenderweise wurde nun für die Verbindungen I durch pharmakologische Untersuchungen gefunden, daß sie sich ebenfalls für eine Anwendung als Heilmittel gegen Störungen der ableitenden Harnwege eignen.

Die Erfindung betrifft daher die Anwendung der Verbindungen der Formel I bei der Behandlung und Prophylaxe der vorstehend aufgeführten Krankheiten, besonders bevorzugt sind dabei solche Krankheiten, bei denen eine Störung der glattmuskulären Kontraktionen der jeweiligen Organe vorli wie beispielsweise Inkontinenz oder Nierenkoliker Eine besondere Bedeutung kommt dabei solchen Verbindungen I zu, deren blutdrucksenkende Eigenschaften weniger stark ausgeprägt sind.

Die Erfindung ist weiterhin gerichtet auf eine Verbindung I zur Anwendung zur Behandlung von Funktionsstörungen der ableitenden Harnwege.

Ganz besonders bevorzugt ist die Verwendung von 3,4-Dihydro-2,2-dimethyl-6-(2-chlorphenylsulfonyl)-trans-4-(2-oxo-1-pyrrolidinyl)-2H-benzo[b]pyran-3-ol zur Herstellung eines Heilmittels gegen Störungen der ableitenden Harnwege.

Die Erfindung umfaßt weiterhin die Verwendung der erfindungsgemäßen Verbindungen bei der Herstellung von Arzneimitteln, die zur Behandlung und Prophylaxe der vorstehend genannten Krankheiten eingesetzt werden.

Die Verbindungen I können nach folgenden Verfahren hergestellt werden:
Indem man
a) Verbindungen der Formel II 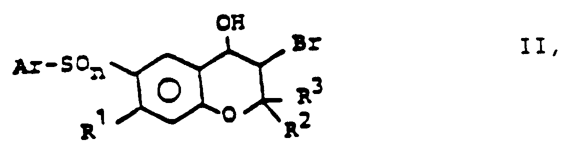 in denen R¹ bis R³ und ArSOₙ wie oben definiert sind umsetzt mit Lactamen der Formel III 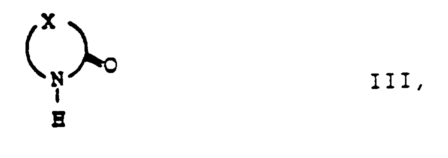
b) Verbindungen der Formel IV 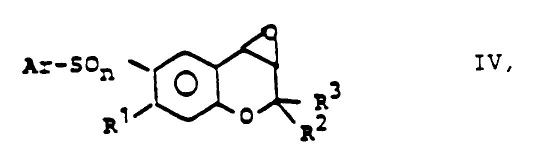 in denen R¹ bis R³ und ArSOₙ wie oben definiert sind, unsetzt mit den Lactamen der Formel III
c) Verbindungen der Formel V 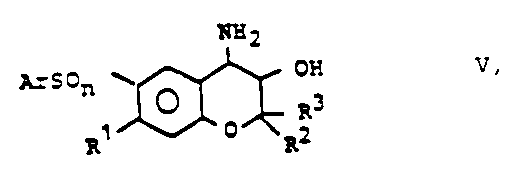 in denen R¹ bis R³ und ArSOₙ wie oben definiert sind, acyliert zu den Verbindungen VI 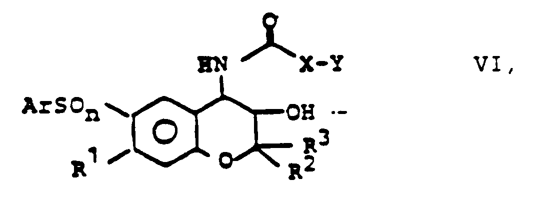 in denen Y eine Fluchtgruppe, wie etwa Chlor oder Brom, ist und R¹ bis R³ und ArSOₙ wie oben definiert sind, und diese zu den Verbindungen I cyclisiert
d) Verbindungen der Formel VII 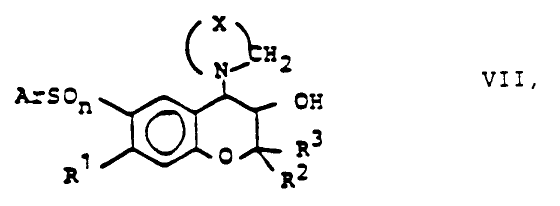 in denen R¹ bis R³ und ArSOₙ wie oben definiert sind, zu den Verbindungen I oxidiert.

Werden die Verbindungen I nach den Methoden a) oder b) hergestellt, so geschieht dies dadurch, daß man die Verbindungen II oder IV in einem geeigneten Lösemittel, vorzugsweise in dipolar aprotischen Lösemitteln wie etwa Dimethylsulfoxid oder THF umsetzt mit den Lactamen III, vorzugsweise unter Einwirkung von Basen, wie etwa Natriumhydrid, K-tert.butylat oder ähnlichen, für Lactam-N-Alkylierungen bekanntermaßen geeigneten Basen. Die Reaktionstemperatur ist dabei im weiten Bereich variierbar; vorzugsweise wird zwischen 0° und Zimmertemperatur gearbeitet oder bei Temperaturen, die leicht oberhalb der Zimmertemperatur liegen können.

Lactame der Formel III sind in vielen Fällen bekannt, oder können leicht nach literaturbekannten Methoden hergestellt werden. Verbindungen II oder IV sind neu. Sie können beispielsweise nach folgendem Syntheseweg hergestellt werden:
Verbindungen der Formel VIII 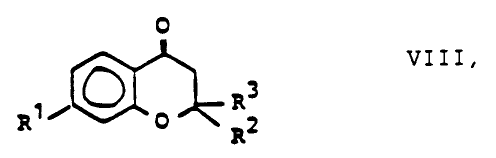
in denen R¹, R² und R³ wie oben definiert sind, werden mit Säurechloriden Ar-SOₙ-Cl in an sich bekannter Weise nach Art der Friedel-Crafts Acylierung umgesetzt zu Verbindungen der Formel IX 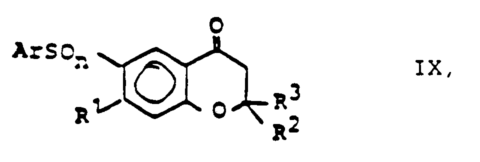
in denen R¹, R², R³ und Ar und n wie oben definiert sind. Diese werden durch Reduktionen unter Standardbedingungen etwa durch NaBH₄ in Methanol umgesetst zu den Verbindungen X 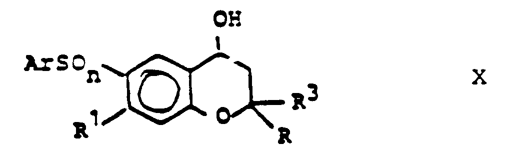
und anschließend einer Wasserabspaltung, etwa durch Pyridin/Phosphoroxychlorid, unterwerfen, wobei Verbindungen der Formel XI entstehen:

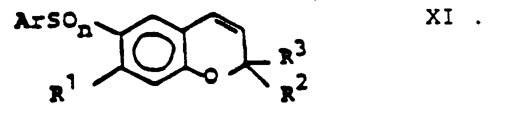

Verbindungen XI lassen sich nun leicht nach Standardmethoden in die Epoxide IV oder die Bromhydrine II umwandeln.

Steht bei dieser Reaktionssequenz R in der Bedeutung von NH₂ oder OH, so sind gegebenenfalls Schutzgruppen wie etwa die Dimethylaminomethylengruppe für NH₂ oder die Acetyl- oder Methylgruppe für die OH-Gruppe notwendig. Diese werden auf geeigneten Stufen, vorzugsweise nach Durchführung die in Verfahren a) oder b) beschriebenen Umsetzungen durch gängige Methoden wieder abgespaltet.

Chromene der Formel XI werden in einigen Fällen in an sich bekannter Weise durch thermisch induzierte Cyclisierung der entsprechenden Propargylether XII 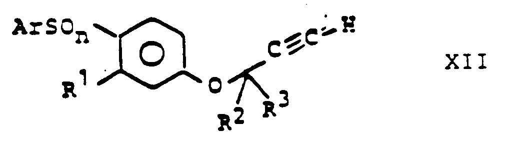
hergestellt. Diese wiederum lassen sich in an sich bekannter Weise aus den Phenolen XIII und den Propargylchloriden XIV herstellen. 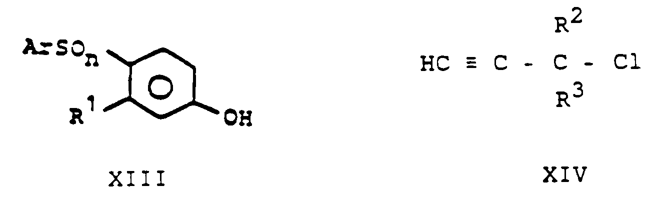
Die Verfahren c) und d) können dann besonders günstig angewandt werden, wenn enantiomerenreine Endprodukte I erwünscht sind. Verbindungen V und VII sind im Gegensatz zu Verbindungen I basisch und damit zur Salzbildung mit organischen Säuren befähigt. Durch Kristallisation mit einer geeigneten, optisch einheitlichen Säure wie etwa (+)-Mandelsäure oder (+)-Milchsäure können sie in an sich bekannter Weise enantiomerenrein erhalten werden und in enantiomerenreine Endprodukte I nach den Verfahren c) und d) umgewandelt werden.

Enantiomerenreine Endprodukte I können aber auch aus racemischen Endprodukten I durch gängige Racematspaltungsmethoden wie etwa die chromatographische Trennung unter Verwendung von chiralen Phasen oder die Derivatisierung der racemischen Produkte mit optisch einheitlichen Säurederivaten (Esterbildung über die 3-Hydroxygruppe des Chromansystems) oder mit optisch einheitlichen Isocyanaten (Carbamatbildung über die 3-Hydroxygruppe) erhalten werden. Die dabei erhaltenen diastereoisomeren Isocyanate oder Ester lassen sich durch gängige Methoden (Kristallisation oder Chromatographie) trennen und unter Abspaltung der optisch aktiven Hilfsgruppe an der 3-OH-Funktion in die optisch einheitlichen Endverbindungen I umwandeln. Als besonders vorteilhaft hat sich hierbei die Trennung der diastereomeren 3-Menthoxyacetate erwiesen.

Die Verbindungen I können wie bereits erwähnt erfindungsgemäß als Mittel zur Behandlung von Störungen der ableitenden Harnwege eingesetzt werden. Arzneimittel, die die Verbindung I enthalten, können dabei oral, parenteral, intravenös, rektal oder durch Inhalation appliziert werden, wobei die bevorzugt Applikationsform von der zu behandelnden Krankheit abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann aufgrund seines Fachwissens geläufig. Neben Lösemitteln, Gelbildnern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen wie Trägerstoffen, Stabilisatoren oder inerten Verdünnungsmitteln vermischt und durch die üblichen Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierungen für die Verabreichung in Form von Aerosolen oder Sprays sind geeignet z. B. Lösungen, Suspensionen oder Emulsionen des Wirkstoffs der Formel I in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindung ab; außerdem auch von Art und Stärke der zu behandelnden Krankheit sowie von Geschlecht, Alter, Gewicht und individueller Ansprechbarkeit des zu behandelndes Säugers. Im Durchschnitt beträgt die empfohlene tägliche Dosis einer Verbindung der Formel I bei einem etwa 75 kg schweren Patienten mindestens 0,1 mg, vorzugsweise mindestens 1 mg, bis maximal 100 mg, vorzugsweise bis maximal 10 mg. Bei akuten Ausbrüchen der Krankheit, etwa bei Asthmaanfällen oder bei Nierenkoliken, können dabei mehrere, z. B. bis zu 4 Einzeldosen pro Tag, erforderlich sein, während zur Prophylaxe auch eine Dosierung ausreichen kann.

Die Verbindungen I können dabei allein oder in Kombination mit anderen Verbindungen appliziert werden
Bei der Verwendung gegen Störungen der ableitenden Harnwege kommen beispielsweise antibakteriell oder analgetisch wirksame Kombinationspartner in Frage.

Die in der folgenden Tabelle zusammengestellten Verbindungen der Formel I sind besonders gut geeignet:
1) 2,2-Dimethyl-3,4-dihydro-7-methoxy-6-(p-chlorphenylsulfonyl)-trans-4-(2-oxo-1-pyrrolidinyl)-2H-benzo[b]pyran-3-ol,
2) 2,2-Dimethyl-3,4-dihydro-6-(p-chlorphenylsulfonyl)-trans-4-(2-oxo-1-piperidinyl)-2H-benzo[b]pyran-3-ol,
3) 2,2-Dimethyl-3,4-dihydro-6-(p-nitrophenylsulfonyl)-trans-4-(2-oxo-1-pyrrolidinyl)-2H-benzo[b]pyran-3-ol,
4) 2,2-Dimethyl-3,4-dihydro-6-(p-cyanophenylsulfonyl)-trans-4-(2-oxo-1-pyrrolidinyl)-2H-benzo[b]pyran-3-ol,
5) 2,2-Dimethyl-3,4-dihydro-6-(p-methoxyphenylsulfonyl)-trans-4-(2-oxo-1-pyrrolidinyl)-2H-benzo[b]pyran-3-ol,
6) 2,2-Dimethyl-3,4-dihydro-6-(p-trifluormethylphenylsulfonyl)-trans-4-(2-oxo-1-pyrrolidinyl)-2H-benzo[b]pyran-3-ol,
7) 2,2-Dimethyl-3,4-dihydro-6-(p-methylsulfonylphenylsulfonyl)-trans-4-(2-oxo-1-pyrrolidinyl)-2H-benzo[b]pyran-3-ol,
8) 2,2-Dimethyl-3,4-dihydro-6-(p-acetylphenylsulfonyl)-trans-4-(2-oxo-1-pyrrolidinyl)-2H-benzo[b]pyran-3-ol,
9) 2,2-Dimethyl-3,4-dihydro-7-methylamino-6-phenylsulfonyl-trans-4-(2-oxo-1-pyrrolidinyl)-2H-benzo[b]pyran-3-ol,
10) 2,2-Dimethyl-3,4-dihydro-7-fluor-6-phenylsulfonyl-trans-4-(2-oxo-1-pyrrolidinyl)-2H-benzo[b]pyran-3-ol,
11) 2,2-Diethyl-3,4-dihydro-7-fluor-6-phenylsulfonyl-trans-4-(2-oxo-1-pyrrolidinyl)-2H-benzo[b]pyran-3-ol,
12) 2,2-Dimethyl-7-chlor-3,4-dihydro-6-phenylsulfonyl-trans-4-(2-oxo-1-pyrrolidinyl)-2H-benzo[b pyran-3-ol,
13) 2,2-Dimethyl-3,4-dihydro-6-(4-chlor-3-methylphenylsulfonyl)-trans-4-(2-oxo-1-pyrrolidinyl)-2H-benzo[b]pyran-3-ol,
14) 2,2-Dimethyl-3,4-dihydro-6-(4-chlorphenylsulfonyl)-trans-(5-oxo-3-thiazolidinyl)-2H-benzo[b]pyran-3-ol,
15) 2,2-Dimethyl-3,4-dihydro-trans-4-(4-methyl-2-oxo-1-piperazinyl)-6-phenylsulfonyl-2H-benzo[b]pyran-3-ol,
16) 2,2-Dimethyl-3,4-dihydro-6-phenylsulfonyl-trans-4-(2-oxo-1-morpholinyl)-2H-benzo[b]pyran-3-ol,
17) 2,2-Dimethyl-3,4-dihydro-6-phenylsulfonyl-trans-4-(5-oxo-3-oxazolinyl)-2H-benzo[b]pyran-3-ol.
18) 3,4-Dihydro-2,2-dimethyl-6-(p-fluorphenylsulfonyl)-trans-4-(2-oxo-1-pyrrolidinyl)-2H-benzo[b]pyran-3-ol,
19) 3,4-Dihydro-2,2-dimethyl-6-(o-fluorphenylsulfonyl)-trans-4-(2-oxo-1-pyrrolidinyl)-2H-benzo[b]pyran-3-ol,
20) 3,4-Dihydro-2,2-dimethyl-6-(3-pyridylsulfonyl)-trans-4-(2-oxo-1-pyrrolidinyl)-2H-benzo[b]pyran-3-ol,
21) 3,4-Dihydro-2,2-dimethyl-6-(2-pyrimidinylsulfonyl)-trans-4-(2-oxo-1-pyrrolidinyl)-2H-benzo[b]pyran-3-ol,
22) 3,4-Dihydro-2,2-dimethyl-6-(2-furylsulfonyl)-trans-4-(2-oxo-1-pyrrolidinyl)-2H-benzo[b]pyran-3-ol.

### Beispiel 1

### 3,4-Dihydro-2,2-dimethyl-7-methoxy-6-(p-tolylsulfonyl)-trans-4-(2-oxo-1-pyrrolidinyl)-2H-benzo[b]pyran-3-ol

4,3 g (0,0097 Mol) 3-Brom-3,4-Dihydro-2,2-dimethyl-7-methoxy-6-(p-tolylsulfonyl)-2H-benzo[b]pyran-4-ol werden in 28 ml Dimethylsulfoxid gelöst, mit 3,5 ml 2-Pyrrolidinon (0,0465 Mol) und 0,78 g Natriumhydrid (80 %ige Suspension in Öl) (0,0325 Mol) versetzt und 3 Stunden bei 40°C gerührt. Man läßt über Nacht stehen, gießt den Ansatz auf Eiswasser, und saugt ab. Der Niederschlag wird aus Isopropanol umkristallisiert. Weiße Kristalle vom Schmp.: 263 - 65°C.

### Herstellung der Ausgangsverbindung:

3-Brom-3,4-dihydro-2,2-dimethyl-7-methoxy-6-(p-tolylsulfonyl)-2H-benzo[b]pyran-4-ol erhält man aus 2,2-Dimethyl-7-methoxy-6-(p-tolylsulfonyl)-2H-chromen und N-Bromsuccinimid in einem 9:1 Gemisch aus Dimethylsulfoxid/H₂O. Schmp.: 200-201°C
2,2-Dimethyl-7-methoxy-6-(p-tolylsulfonyl)-2H-chromen erhält man aus 2,2-Dimethyl-7-methoxy-6-(p-tolylsulfonyl)-chroman-4-ol mit Phosphoroxychlorid/Pyridin in Benzol.
Schmp.: 132 - 33°C
2,2-Dimethyl-7-methoxy-6-(p-tolylsulfonyl)-chroman-4-ol erhält man aus 2,2-Dimethyl-7-methoxy-6-(p-tolylsulfonyl)chroman-4-on mit NaBH₄ in Ethanol.
Schmp.: 196 - 97°C
2,2-Dimethyl-7-methoxy-6-(p-tolylsulfonyl)chroman-4-on erhält man aus 2,2-Dimethyl-7-methoxy-chroman-4-on und p-Toluolsulfonsäurechlorid in Gegenwart von Aluminiumchlorid in Methylenchlorid.
Schmp.: 221 - 23°C.

### Beispiel 2

### 3,4-Dihydro-2,2-dimethyl-7-methoxy-6-(p-tolylsulfonyl)-trans-4-(2-oxo-1-piperidinyl)-2H-benzo[b]pyran-3-ol

5 g (0,011 Mol) 3-Brom-3,4-dihydro-2,2-dimethyl-7-methoxy-6-(p-tolylsulfonyl)-2H-benzo[b]pyran-4-ol werden in 32 ml Dimethylsulfoxid gelöst und mit 4,9 g Valerolactam (0,0526 Mol) sowie 0.8 g (0,033 Mol)NaH, 80 %ige Suspension in Öl, versetzt und 5 Stunden bei 40°C gerührt. Man gießt den Ansatz auf Eiswasser und saugt ab. Der Rückstand wird mehrfach mit Methanol ausgekocht.
Weiße Kristalle vom Schmp.: 261 - 63°C

### Beispiel 3

### 3,4-Dihydro-2,2-dimethyl-7-methoxy-6-phenylsulfonyl-trans-4-(2-oxo-1-pyrrolidinyl)-2H-benzo[b]pyran-3-ol

Die Verbindung wird analog Beispiel 1 aus 3-Brom-3,4-dihydro-2,2-dimethyl-7-methoxy-6-phenylsulfonyl-2H-benzo[b]pyran-4-ol hergestellt.
Weiße Kristalle vom Schmp.: 227 - 29°C

### Trennung der Antipoden, Beispiel 3a

1,075 g (0,0025 Mol) (±)-3,4-Dihydro-2,2-dimethyl-7-methoxy-6-phenylsulfonyl-trans-4-(2-oxo-1-pyrrolidinyl)-2H-benzo[b]pyran-3-ol werden in 5 ml 1,2-Dichlorbenzol gelöst, mit 0,9 g S(-)-1-Phenylethylisocyanat versetzt und ca 12 h bei 140°C gerührt. Der komplette Ansatz wird anschließend an Kieselgel mit dem Laufmittelsystem Toluol/Essigester 1:1 chromatographiert. Das langsamer laufende diastereomere Carbamat kann angereichert werden und durch Kristallisation aus Toluol rein erhalten werden (Schmp. 243-245°C). Durch Verseifung mit NaOH in EtOH bei 80°C erhalt man das (+)-3,4-Dihydro-2,2-dimethyl-7-methoxy-6-phenylsulfonyl-trans-4-(2-oxo-1-pyrrolidinyl)-2H-benzo[b]pyran-3-ol mit dem Schmp.: 209-211°C und [α]_{D} = + 109° (c= 0,28; CHCl₃)

### Herstellung des Ausgangsmaterials:

3-Brom-3,4-dihydro-2,2-dimethyl-7-methoxy-6-phenylsulfonyl-2H-benzo[b]pyran-4-ol erhält man aus 2,2-Dimethyl-7-methoxy-6-phenylsulfonyl-2H-chromen und N-Bromsuccinimid in einem 9:1 Dimethylsulfoxid/H₂O Gemisch. Schmp.: 202 - 203°C.

2,2-Dimethyl-7-methoxy-6-phenylsulfonyl-2H-chromen erhält man aus 2,2-Dimethyl-4-hydroxy-7-methoxy-6-phenylsulfonylchromen mit Pyridin/Phosphoroxychlorid in Benzol.
Schmp: 140 - 41°C.

2,2-Dimethyl-4-hydroxy-7-methoxy-6-phenylsulfonylchroman erhält man aus 2,2-Dimethyl-7-methoxy-6-phenylsulfonylchroman-4-on mit Natriumborhydrid in Methanol.
Schmp.: 146 - 147°C.

2,2-Dimethyl-7-methoxy-6-phenylsulfonylchroman-4-on erhält man aus Phenylsulfonylchlorid,
2,2-Dimethyl-7-methoxychroman-4-on und Aluminiumchlorid in Methylenchlorid.
Schmp.: 223 - 25°C

### Beispiel 4

### 3,4-Dihydro-2,2-dimethyl-6-(4-methylphenylsulfonyl)-trans-4-(2 -oxo-1-pyrrolidinyl)-2H-benzo[b]pyran-3-ol

Zu 8,2 g (0,02 Mol) 3-Brom-3,4-dihydro-2,2-dimethyl-6-(4-methylphenylsulfonyl)-2H-benzo[b]pyran-4-ol in 30 ml Dimethylsulfoxid werden 0,75 g (0,025 Mol) 80 %iges NaH eingetragen. Nach einstündigem Rühren bei 20° werden weitere 0,75 g (0,025 Mol) 80 % NaH und 1,9 ml (0,025 Mol) 2-Pyrrolidon zugefügt und 45 Minuten bei 40° und 6 Stunden bei 20° gerührt. Nach Eintragen in Eiswasser wird der Niederschlag abgesaugt, getrocknet und mehrfach aus Methanol umkristallisiert.
Kristalle vom Schmp.: 242 - 243°C.

### Herstellung des Ausgangsmaterials

### 3-Brom-3,4-dihydro-2,2-dimethyl-6-(4-methylphenylsulfonyl)-2H-benzo[b]pyran-4-ol

Zu 12,6 g (0,04 Mol) 2,2-Dimethyl-6-(4-methylphenylsulfonyl)-chromen in einer Lösung aus 70 ml Dimethylsulfoxid und 1,4 ml Wasser werden unter Kühlung (Isopropanol/Trockeneis) bei ungefähr 15°C 14,2 g (0,08 Mol) frisch umkristallisierteS N-Bromsuccinimid eingetragen. Die Temperatur steigt vorübergehend auf 27°. Es wird auf 20°C abgekühlt und nach einstündigem Rühren in Eis/Essigester eingetragen. Die Essigesterphase wird mehrfach mit Wasser gewaschen und über Na₂SO₄ getrocknet. Beim Einengen kristallisiert das Bromhydrin-Derivat.
Kristalle vom Schmp.: 141 - 142°C.

### Beispiel 5

### 3,4-Dihydro-2,2-dimethyl-6-phenylsulfonyl-trans-4-(2-oxo-1-pyrrolidinyl)-2H-benzo[b]pyran-3-ol

Zu einer Suspension von 0,6 g (0,02 Mol) 80 %igem NaH in 10 ml DMSO wird eine Lösung aus 6,3 g (0,02 Mol) 3,4-Dihydro-2,2-dimethyl-3,4-epoxy-6-phenylsulfonyl-2H-benzo[b]pyran in 20 ml DMSO bei 20° zugetropft. Anschließend werden 2,3 ml (0,03 Mol) 2-Pyrrolidinon zugefügt und 1 Stunde bei 45° gerührt. Nach Stehen über nacht bei 20° wird in Eiswasser eingetragen. Der Niederschlag wird abgesaugt, neutral gewaschen, getrocknet und an Kieselgel mit Methylenchlorid/Methanol 19:1 chromatographiert. 30 ml-Fraktionen werden aufgefangen. Die Fraktionen 12-25 werden eingeengt und der Rückstand wird aus Acetonitril umkristallisiert.
Schmp.: 201-202°

### Herstellung des Ausgangsmaterials:

3,4-Dihydro-2,2-dimethyl-3,4-epoxy-6-phenylsulfonyl-2H-benzo[b]pyran erhält man aus 3-Brom-3,4-dihydro-2,2-dimethyl-6-phenylsulfonyl-2H-benzo[b]pyran-4-ol mit NaH in DMSO.
Schmp.: 103-105°
3-Brom-3,4-dihydro-2,2-dimethyl-6-phenylsulfonyl-2H-benzo[b]pyran-4-ol erhält man aus 2,2-Dimethyl-6-phenylsulfonyl-2H-chromen und N-Bromsuccinimid in einem 9:1 Dimethylsulfoxid/H₂O Gemisch
Schmp.: 126°
Das 2,2-Dimethyl-6-phenylsulfonyl-2H-chromen mit dem Schmp.: 70-71° wurde nach bekannten Methoden aus 4-Phenylsulfonylphenyl-1,1 -dimethylpropargylether hergestellt. Diesen Ether erhielt man in ebenfalls bekannter Weise aus 4-Phenylsulfonylphenol und 3-Methyl-3-chlorbutin.

### (+)-3,4-Dihydro-2,2-dimethyl-6-phenylsulfonyl-4-(2-oxo-1-pyrrolidinyl)-2H-benzo[b]pyran-3-ol (Beispiel 5a)

(±)-3,4-Dihydro-2,2-dimethyl-6-phenylsulfonyl-4-(2-oxo-1-pyrrolidinyl)-2H-benzo[b]pyran-3-ol wird nach Standardmethoden mit (-)Menthoxyessigsäurechlorid verestert.
Die diastereomeren Ester werden auf einer Kieselgelsäule mit Methylenchlorid/Essigester (9:1) getrennt und mit alkoholischer Natriumethylatlösung unter Rühren bei 20° verseift. Nach Verdünnen mit kaltem Wasser wird der Niejerschlag abgesaugt und neutral gewaschen und mit Äther verrieben.
(+)-3,4-Dihydro-2,2-dimethyl-6-phenylsulfonyl-4-(2 -oxo-1-pyrrolidinyl)-2H-benzo[b]pyran-3-ol
Schmp.: 122-123° [α]_{D} = + 39,5° (c= 1 , Ethanol)

### Beispiel 6

### 6-(4-Chlorphenylsulfonyl)-3,4-dihydro-2,2-dimethyl-7-methoxy-trans-4-(2-oxo-1-pyrrolidinyl)-2H-benzo[b]pyran-3-ol

Die Verbindung wird analog Beispiel 1 aus 3-Brom-6-(4-Chlorphenylsulfonyl)-3,4-dihydro-2,2-dimethyl-7-methoxy-2H-benzo[b]pyran-4-ol hergestellt.
Weiße Kristalle mit Schmp.: 260-262°C

### Herstellung der Ausgangsverbindungen:

Analog Beispiel 1:
3-Brom-6-(4-Chlorphenylsulfonyl)-3,4-dihydro-2,2-dimethyl-7-methoxy-2H-benzo[b]pyran-4-ol mit Schmp.: 175-177°C
6-(4-Chlorphenylsulfonyl)-2,2-dimethyl-7-methoxy-chromen mit Schmp.: 142-143°C

### Beispiel 7

### 6-(4-Bromphenylsulfonyl)-3,4-dihydro-2,2-dimethyl-7-methoxy-trans-4-(2-oxo-1-pyrrolidinyl)-2H-benzo[b]pyran-3-ol

Analog Beispiel 1 aus 3-Brom-6-(4-Bromphenylsulfonyl)-3,4-dihydro-2,2-dimethyl-7-methoxy-2H-benzo[b]pyran-4-ol.
Weiße Kristalle vom Schmp.: 281-282°C.

### Beispiel 8

### 3,4-Dihydro-2,2-dimethyl-7-methoxy-6-(4-methoxyphenylsulfonyl)-trans-4-(2-oxo-1-pyrrolidinyl)-2H-benzo[b]pyran-3-ol

Die Verbindung wird analog Beispiel 1 aus 3-Brom-3,4-dihydro-2,2-dimethyl-7-methoxy-6-(4-methoxyphenylsulfonyl)-2H-benzo[b]pyran-4-ol hergestellt und hat einen Schmp. von 286-287°C.

### Beispiel 9

### 3,4-Dihydro-2,2-dimethyl-7-methoxy-6-(2-thienylsulfonyl)-trans-4-(2-oxo-1-pyrrolidinyl)-2H-benzo[b]pyran-3-ol

Analog Beispiel 1 aus 3-Brom-3,4-dihydro-2,2-dimethyl-7-methoxy-6-(2-thienylsulfonyl)-2H-benzo[b]pyran-4-ol,
Schmp.: 135-136°C.

### Beispiel 10

### 3,4-Dihydro-2,2-dimethyl-7-ethoxy-6-phenylsulfonyl-trans-4-(2-oxo-1-pyrrolidinyl)-2H-benzo[b]pyran-3-ol

Analog Beispiel 1 aus 3-Brom-3,4-dihydro-2,2-dimethyl-7-ethoxy-6-phenylsulfonyl-2H-benzo[b]pyran-4-ol,
Schmp: 197-198°C.

### Beispiel 11

### 3,4-Dihydro-2,2-dimethyl-7-methoxy-6-phenylsulfonyl-trans-4-(2-oxo-1-piperidinyl)-2H-benzo[b]pyran-3-ol

Analog Beispiel 2 aus 3-Brom-3,4-dihydro-2,2-dimethyl-7-methoxy-6-phenylsulfonyl-2H-benzo[b]pyran-4-ol,
Weiße Kristalle vom Schmp.: 157-158°C.

### Beispiel 12

### 6-(4-Cyanphenylsulfonyl)-3,4-dihydro-2,2-dimethyl-trans-4-(2-oxo-1-pyrrolidinyl)-2H-benzo[b]pyran-3-ol

Analog Beispiel 1 aus 3-Brom-6-(4-cyanphenylsulfonyl)-3,4-dihydro-2,2-dimethyl-2H-benzo[b]pyran-4-ol,
Weiße Kristalle vom Schmp.: 234-235°C.

### Herstellung des Ausgangsmaterials:

3-Brom-6-(4-cyanphenylsulfonyl)-3,4-dihydro-2,2-dimethyl-2H-benzo[b]pyran-4-ol erhält man wie unter Beispiel 3 beschrieben aus 6-(4-Cyanphenylsulfonyl)-2,2-dimethyl-3-chromen.
Schmp.: 157-158°C.

### Beispiel 13

### 3,4-Dihydro-2,2-dimethyl-6-(2-methoxyphenylsulfonyl)-trans-4-(2-oxo-1-pyrrolidinyl)-2H-benzo[b]pyran-3-ol

Analog Beispiel 1 aus 3-Brom-3,4-dihydro-2,2-dimethyl-6-(2-methoxyphenylsulfonyl)-2H-benzo[b]pyran-4-ol.
Weiße Kristalle vom Schmp.: 196-198°C.

### Beispiel 14

### 3,4-Dihydro-2,2-dimethyl-6-(2-methylphenylsulfonyl)-trans-4-(2-oxo-1-pyrrolidinyl)-2H-benzo[b]pyran-3-ol

Analog Beispiel 1. Weiße Kristalle vom Schmp.: 214-216°C.

### Beispiel 15

### 3,4-Dihydro-2,2-dimethyl-6-(2-chlorphenylsulfonyl)-trans-4-(2-oxo-1-pyrrolidinyl)-2H-benzo[b]pyran-3-ol

Analog Beispiel 1. Weiße Kristalle vom Schmp.: 85-87°C

### Beispiel 16

### Herstellung von 3,4-Dihydro-2,2-dimethyl-6-phenylsulfonyl-trans-4-(2-oxo-1-pyrrolidinyl)-2H-benzo[b]pyran-3-ol (Verbindung von Beispiel 5 nach Verfahrensvariante c)

Eine Lösung von 3-Brom-3,4-dihydro-2,2-dimethyl-6-phenylsulfonyl-2H-benzo[b]pyran-4-ol in Ethanol wird 8 Stunden bei 50° im Autoklaven bei einem Druck von 8 bar NH₃ geschüttelt. Nach dem Abkühlen wird bis zur Trockene eingeengt und aus Essigester umkristallisiert. Man erholt das 4-Amino-3,4-dihydro-2,2-dimethyl-6-phenylsulfonyl-2H-benzo[b]pyran-3-ol vom Schmp.: 160-163°C, das sofort einer Acylierung mit 4-Chlorbuttersäurechlorid unterworfen wird. Hierzu wird die Substanz sowie das Säurechlorid in CH₂Cl₂ und im Zweiphasengemisch mit 2N Natronlauge 24 Stunden bei Zimmertemperatur gerührt. Nach üblicher Aufarbeitung erhält man das 4-(4-Chlorbutyrylamino)-3,4-dihydro-2,2-dimethyl-6-phenylsulfonyl-2H-benzo[b]pyran-4-ol vom Schmp.: 155-157°C. Die Cyclisierung zur Titelverbindung erfolgt durch Lösung der Substanz in Tetrahydrofuran, Zugabe einer stöchiometrischen Menge 80 %igem NaH-Suspension inÖl undRühren des Gemisches bei Zimmertemperatur über 24 Stunden. Das Endprodukt ist mit dem nach Verfahren b) gewonnenen Produkt identisch. Schmp.: 200-201°C. Wird das 4-Amino-3,4-dihydro-2,2-dimethyl-6-phenylsulfonyl-2H-benzo[b]pyran-3-ol einer Racematspaltung unterworfen, so kann aus seinem (+)-Enantiomer das reine (+)-Enantiomer aus Beispiel 5a mit den dort angegebenen Daten erhalten werden.

### Beispiel 17

### 3,4-Dihydro-2,2-dimethyl-6-phenylsulfoxy-trans-4-(2-oxo-1-pyrrolidinyl)-2H-benz[b]pyran-3-ol

Analog Beispiel 1. Schmp.: 211 - 212°C

### Pharmakologische Daten

Ableitende Harnwege
Beeinflussung der KCl-induzierten rhythmischen Kontraktionen am Meerschweinchen-Ureteren in vitro

### Methode:

Männliche Meerschweinchen wurden durch Nackenschlag und Entbluten aus den Carotiden getötet. Die beiden Ureteren wurden sofort entnommen, wobei der Bereich nahe dem Nierenkelch wegen der dort vorhandenen Schrittmacher-Aktivität gemieden wurde. 2 cm lange Stücke wurden zunächst in einer Petrischale, die Tyrode-Lösung enthielt, von Bindegewebe befreit und dann jeweils mit einer Vorspannung von 4,9 mN in ein 25 ml-Organbad (Fa. Rhema Labortechnik, Hofheim) gehängt. Das Organbad enthielt eine auf 37°C gehaltene und mit Carbogen (95 % O₂, 5 % CO₂) durchperlte Tyrodelösung mit folgender Zusammensetzung (mmol/l): NaCl 137, KCl 2,68, MgSO₄ 1,05, CaCl₂ 1,8, NaH₂PO₄ 0,41, NaHCO₃ 11,9, Glucose 5,55.

Die Kontraktionen wurden isometrisch gemessen unter Verwendung von Gould/Statham UC2 Gebern. Nach einer Äquilibrierungszeit von mindestens 15 Minuten wurde KCl in das Organbad zugegeben, wobei eine Konzentration von 4 x 10⁻² mol/l erreicht wurde. Der Agonist wurde 2 Minuten im Bad gelassen, wobei phasische Kontraktionen auftraten, ohne einen nennenswerten Anstieg der Grundspannung zu bewirken.

Danach wurde 1 Minute ausgespült, woraufhin die rhythmischen Kontraktionen sofort aufhörten. Nach einem zweiten Durchgang von Agonistgabe und Spülvorgang wurde die Prüfsubstanz (Benzopyran-Derivat) in das Organbad gegeben (in Form einer Lösung in 0,1 ml Ethanol, die Endkonzentrationen betrugen in allen Fällen 10⁻⁷ mol/l) und eine Einwirkungszeit von einer Minute abgewartet, bevor KCl zugesetzt wurde. An den folgenden Spülvorgang schlossen sich zwei abschließende Agonistgabe/Spülgänge an. Von den jeweils zweiminütigen Perioden unter KCl-Einwirkung wurden folgende Parameter bestimmt: 1. mittlere Kontraktionskraft, 2. Frequenz der Kontraktionen und 3. Produkt aus mittlerer Kontraktionskraft und Frequenz.

Ausschlußkriterien waren mittlere Kontraktionskräfte unter 4 mN oder Frequenzen unter 2/min bei mehr als einer der vier Perioden, bei denen nur der Agonist KCl im Bad vorhanden war. Ausgewertet wurde die prozentuale Hemmung unter Prüfsubstanz im Vergleich zum gemittelten Wert aus den beiden Vorläufen.

Neben dem arithmetischen Mittelwert (x) wurde der Standardfehler des Mittelwerts (SEM) errechnet.

### Ergebnisse:

- n =: Anzahl der Ureteren

| Verbindung | mittlere Kontraktionskraft k (%) | Frequenz v (%) | k · v (%) | n |
|---|---|---|---|---|
| 3,4-Dihydro-2,2-dimethyl-6-(2-chlorphenylsulfonyl)-trans-4-(2-oxo-1-pyrrolidinyl)-2H-benzo[b]pyran-3-ol Beispiel 15 | 68 ± 11 | 67 ± 14 | 85 ± 7 | 4 |

- Literatur:: R. Schiantarelli und W. Murmann, Arzneimittel-Forschung/Drug Research 30, 1102 - 1109 (1980)

## Patentansprüche

1. Verwendung einer Verbindung I 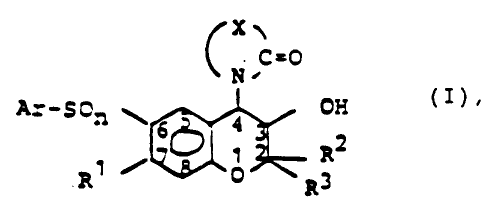 in welcher
R¹ für H, OH, (C₁-C₂)-Alkoxy, (C₁-C₂)-Alkyl oder NR⁴R⁵ steht, wobei R⁴ und R⁵ gleich oder verschieden sind und für H, (C₁-C₂)Alkyl oder (C₁-C₃)-Alkylcarbonyl stehen,
R²,R³ gleich oder verschieden sind und für Alkyl mit 1-4 C-Atomen stehen,
Ar für ein aromatisches oder heteroaromatisches System seht, das unsubstituiert oder durch 1 bis 3 gleiche oder verschiedene Reste (C₁-C₂)-Alkyl, (C₁-C₂)Alkoxy, Halogen, Trifluormethyl, CN, NO₂, CO-(C₁-C₂)Alkyl oder SOₘ-(C₁-C₂)-Alkyl mit m= 1 oder 2 substituiert ist,
n für 1 oder 2 steht,
X für eine Kette (CH₂)ᵣ steht, die durch ein Heteroatom 0, S oder NR⁶ unterbrochen sein kann, wobei R⁶ H oder (C₁-C₄)-Alkyl bedeutet und r für die Zahlen 2, 3, 4 oder 5 steht,
zur Herstellung eines Heilmittels gegen Störungen der ableitenden Harnwege.

2. Verbindung I zur Anwendung zur Behandlung von Funktionsstörungen der ableitenden Harnwege.

3. Verwendung einer Verbindung I nach Anspruch 1, bei der R¹ bis R³ und ArSOₙ wie in Anspruch 1 definiert sind, und X (CH₂)ᵣ mit r= 3 oder 4 bedeutet.

4. Verwendung nach Anspruch 1, dadurch gekennzeichnet, daß
R¹ bis R³ die oben erwähnten Bedeutungen haben,
Ar für unsubstituiertes oder wie nach Anspruch 1 substituiertes Phenyl steht, und
X (CH₂)ᵣ mit r= 3 oder 4 bedeutet.

5. Verwendung nach Anspruch 1, dadurch gekennzeichnet, daß
R¹ Wasserstoff bedeutet,
R² und R³ für (C₁-C₂)-Alkyl stehen,
Ar Phenyl bedeutet, das unsubstituiert oder durch (C₁-C₂)-Alkyl, (C₁-C₂)-Alkoxy oder Halogen einfach substituiert ist,
n 2 bedeutet, und
X (CH₂)ᵣ mit r= 3 oder 4 bedeutet.

6. Verwendung einer Verbindung I nach Anspruch 1, dadurch gekennzeichnet, daß
R¹ Wasserstoff bedeutet,
R² und R³ für (C₁-C₂)-Alkyl stehen,
X (CH₂)₃ bedeutet,
Ar C₆H₄Cl und
n 2 bedeuten,
zur Herstellung eines Heilmittels gegen Störungen der ableitenden Harnwege.

7. Verwendung nach Anspruch 1, dadurch gekennzeichnet, daß
R¹ Wasserstoff bedeutet,
R² und R³ für (C₁-C₂)-Alkyl stehen,
Ar Phenyl bedeutet,
n 2 bedeutet, und
X (CH₂)₃ bedeutet.

8. Verwendung von 3,4-Dihydro-2,2 dimethyl-6-(2-Chlorphenylsulfonyl)-trans-4-(2-oxo-1-pyrrolidinyl)-2H-benzo[b]pyran-3-ol zur Herstellung eines Heilmittels gegen Störungen der ableitenden Harnwege.
